# EUROPEAN PATENT APPLICATION

(11) **EP 2 028 417 A1**
(43) Date of publication of application: **25.02.2009**
(21) Application number: 07745052.6
(22) Date of filing: 12.06.2007
(51) Int. Cl.: F21V 33/00, A61L 9/00, A61L 9/01, B01J 35/02, F21V 3/04, F21Y 103/00

(54) **LIGHTING EQUIPMENT EXCELLING IN AIR PURIFICATION**

(30) Priority: 13.06.2006 JP 2006163935
(71) Applicant: OSAKA Titanium Technologies Co., Ltd., Amagasaki-shi, Hyogo 660-8533 (JP)
(72) Inventor: OGASAWARA, Tadashi, Amagasaki-shi Hyogo 660-8533 (JP); MATSUO, Toshiaki, Amagasaki-shi Hyogo 660-8533 (JP); SHIMOSAKI, Shinji, Amagasaki-shi Hyogo 660-8533 (JP); AZUMA, Kazuomi, Amagasaki-shi Hyogo 660-8533 (JP); MITANI, Yoji, Amagasaki-shi Hyogo 660-8533 (JP)
(74) Representative: Jackson, Martin Peter
(86) International application number: PCT/JP2007/061765
(87) International publication number: WO 2007/145184

(57) **Abstract**

A full spectrum light emitting lamp and one or more translucent base bodies surrounding the lamp are disposed in the lighting apparatus, the one or more translucent bas bodies having a photocatalytic reaction layer which bears a photocatalyst made of a titanium dioxide thin film therein, or the one or more translucent base bodies having the photocatalytic reaction layer which bears the photocatalyst made of the titanium dioxide thin film therein and having infrared light-absorbing functions, and a space through which air can flow is formed between the lamp and the translucent base body. Therefore, an ultraviolet light, a visible light and an infrared light can effectively be utilized according to characteristics such as an air purifying function of the ultraviolet light, a lighting function of the visible light, and a heating function of the infrared light to thereby save optical energy emitted from the lamp. Further, forced convection is generated in the air flow space by a heating effect of the infrared light emitted from the lamp in addition to a heating effect of the optical energy emitted from the lamp, so that the air can sufficiently be ventilated to promote an air purifying effect.

## Description

### TECHNICAL FIELD

The present invention relates to a lighting apparatus, more particularly, to a lighting apparatus having an excellent air purifying property, in which a lamp having a wide wavelength band can be used as a light source and optical energy emitted from the lamp can be utilized without waste.

### BACKGROUND ART

Recently, high attention is paid on photocatalysis of fine particles of an optical semiconductor typified by a titanium dioxide, in particular, on strong oxidation catalysis of the titanium dioxide.

Titanium dioxides are widely used as white paint and pigments for cosmetics. On the other hand, since effects such as dirt decomposition, odor elimination, deodorizing, antibacterial activity, sterilization, and harmful material removal are obtained by their strong oxidizing power, titanium dioxides are applied to products of wide variety of fields such as construction materials, lighting products, and air conditioners. Therefore, for example, titanium dioxides are used for antibacterial glasses and sanitary tiles in the case where a titanium dioxide photocatalyst is to be applied to the construction materials, and titanium dioxides are used for a lighting apparatus having an air purifying function in the case where a titanium dioxide photocatalyst is to be as applied to the lighting product.

In the case where titanium dioxides are used for a lighting apparatus having an air purifying function, its structure is configured so that photocatalysis of titanium dioxides are conducted on an outer surface of a fluorescent lamp. The fluorescent lamp is a light source in which a fluorescent material converts ultraviolet light generated by an arc discharge in a low-pressure mercury vapor in a glass tube into visible light.

That is, in a lighting apparatus in which an outer surface of a fluorescent lamp bears a titanium dioxide photocatalyst, a fluorescent material converts ultraviolet light into visible light, and the titanium dioxide photocatalyst is activated by the ultraviolet light transmitted through the fluorescent material, thereby exerting a strong oxidizing power. Thus, ambient air contacts the activated photocatalyst to oxidize and remove air-pollutants contained in ambient air.

However, there are some problems in the lighting apparatus in which the outer surface of the fluorescent lamp bears the titanium dioxide photocatalyst. The fluorescent material used in the fluorescent lamp has a short life since the fluorescent material deteriorates in fluorescent property every time it repeats an excited state and a ground state, and the fluorescent lamp is probably broken by a shock and the like since the fluorescent lamp comprises a glass tube. Thus, in the case where the fluorescent lamp is discarded and disposed after its life-span or due to damages, the borne photocatalyst is simultaneously discarded to thereby waste the photocatalyst.

A ultraviolet light activating a titanium dioxide photocatalyst has insufficient light intensity since the ultraviolet light is transmitted through the fluorescent material coated on an inner surface of the glass tube that envelops ambient space of a light source. Therefore, the photocatalyst hardly exerts the sufficient air purifying function. In order to solve the problems, various types of apparatus are proposed in order to effectively utilize the optical energy and the photocatalyst.

For example, Japanese Patent Application Publication No. 2003-151343 discloses a lighting apparatus having an air purifying function. The lighting apparatus has a structure in which a translucent cover body envelops an ultraviolet luminescent material, a function layer on which the fluorescent material and the photocatalyst are borne is formed on an inner surface of the cover body, and a space is formed between the cover body and the outer surface of the ultraviolet luminescent material. Therefore, the lighting function and the air purifying function are exerted without wasting the ultraviolet energy emitted from the ultraviolet luminescent material.

Japanese Patent Application Publication No. 2003-151343 proposes ventilation in which the air in the space is ventilated by forced ventilation with a blower or the like and by natural ventilation utilizing convection generated by heat emitted from the ultraviolet luminescent material.

However, in case of the natural convection, that is, in the case where the heat emitted from the ultraviolet luminescent material is utilized, the generation of the convection is restricted to the proximity of the ultraviolet luminescent material, and the convection is not effectively generated near a base body having the photocatalysis effect. Therefore, the efficient air purifying effect is hardly exerted. In case of the forced ventilation with the blower or the like, since an extra electric power is required, the energy is not effectively utilized.

In a method disclosed in Japanese Patent Application Publication No. 2003-151343, in the case where the base body is discarded after the life-span of the fluorescent material, the borne photocatalyst is wasted since the photocatalyst is simultaneously discarded.

Japanese Patent Application Publication No. 2004-000663 discloses an air purifying apparatus including a base body which bears the photocatalyst made of a titanium dioxide thin film and has a photocatalysis reaction surface contacting the air, and a light-emitting diode which mainly emits predetermined visible light and ultraviolet light having a wavelength of 360 to 400 nm. The air purifying apparatus can be applied to a place which is not illuminated with light of the sun, and further, consumes low power and emits color light.

In a method disclosed in Japanese Patent Application Publication No. 2004-000663, since an indoor air is purified by utilizing the natural convection, a lengthy time is required to purify the whole of the indoor air. Therefore, a method for purifying the air for a relatively short time is disclosed in which a purifying apparatus is formed such that a flow path through which the air can flow into and out of the purifying apparatus is formed, the air is caused to flow through the flow path by a fan or the like, and the photocatalysis reaction base body is disposed within the flow path.
However, in the method, because an extra electric power is required to operate the fan, electric power saving which is of an advantage of the light-emittingdiode is hardlyutilized.

Thus, these problems should be solved to develop a lighting apparatus in which optical energy emitted from a light source can effectively be utilized and an air purifying function borne by the titanium dioxide photocatalyst can be exerted.

### DISCLOSURE OF THE INVENTION

As described above, the problem that the ultraviolet light transmitted through the fluorescent material has the weak light intensity and the problem that the air is insufficiently ventilated near the base body having the photocatalysis effect in the ventilation in which the natural convection is utilized should be solved in order to develop the lighting apparatus in which the air purifying function borne by the titanium dioxide photocatalyst can be exerted.

Thus, the excellent air purifying effect borne by the titanium dioxide is not exerted, in the case where the ultraviolet light has the weak light intensity, or where the air is insufficiently ventilated near the base body having the photocatalysis effect.

In view of the foregoing problems, an object of the present invention is to provide a lighting apparatus having an excellent air purifying property, in which a lamp having a wide wavelength band can be used and the air near the base body having the photocatalysis effect can be ventilated sufficiently.

From the perspective that the lighting apparatus having the excellent air purifying property can be developed by the use of the light source and translucent material which are economical and easily available, the present inventors made various studies on a method for exerting the excellent air purifying effect borne by the titanium dioxide and for reducing the waste of the optical energy emitted from the light source. As a result, the inventors obtained the following new findings (a) to (c).

(a) The effects characterized by wavelengths, for example, the air purifying effect of the ultraviolet light, the lighting effect of the visible light, and the heating effect of the infrared light, can effectively exerted in order to utilize the optical energy emitted from the lamp without waste.

(b) In order to sufficiently ventilate near the base body having the photocatalysis effect, the light is transmitted through the thin film or the translucent base body which can convert the light into thermal energy, and whereby the convection is forcedly generated by the heat emitted from the surfaces of the thin film and translucent base body.

(c) When the lamp which is of the light source is surrounded by the plural translucent base bodies, an outermost base body (hereinafter also referred to as "external cylinder") is utilized for decoration and advertisement, and a base body located inside the external cylinder (hereinafter also referred to as "internal cylinder") can bear the photocatalyst. Therefore, it is not necessary to discard the photocatalyst in replacing or discarding the external cylinder.

The present invention is completed based on the findings, and is summarized in the lighting apparatus having the excellent air purifying property described in (1) to (8) below. (1) A lighting apparatus having an excellent air purifying property is characterized in that a full spectrum light emitting lamp and one or more translucent base bodies surrounding the lamp are disposed in the lighting apparatus, the one or more translucent base bodies having a photocatalytic reaction layer which bears a photocatalyst made of a titanium dioxide thin film therein, or the one or more translucent base bodies having the photocatalytic reaction layer which bears the photocatalyst made of the titaniumdioxide thin film therein and having infrared light-absorbing functions, and a space is formed between the lamp and the translucent base body to enable air to flow therethrough.

(2) In the lighting apparatus described in (1), one or more translucent base bodies having the infrared light-absorbing functions are desirably disposed so as to surround the lamp since the generation of convection is promoted in the air flow space.

(3) In the lighting apparatus described in (1) and (2), the lamp desirably emits light which is included in at least ultraviolet spectral and visible spectral wavelength ranges, or the lamp desirably emits light which is included in at least infrared spectral and visible spectral wavelength ranges. Further, the lamp desirably emits light which is included in ultraviolet spectral, visible spectral and infrared spectral wavelength ranges.

(4) In the lighting apparatus described in (1) to (3), the photocatalytic reaction layer can exert photocatalysis activity by an ultraviolet light in the light emitted from the lamp to thereby utilize the ultraviolet light efficiently. Similarly, the photocatalytic reaction layer can exert photocatalysis activity by an ultraviolet light and a visible light in the light emitted from the lamp to thereby supplement the ultraviolet intensity by the visible light.

(5) In the lighting apparatus described in (1) to (4), the infrared light-absorbing function is exerted by an infrared light-absorbingmaterial applied on a surface of the translucent base body to thereby promote the generation of convection in the air flow space. In addition, the infrared light-absorbing function is exerted by a function borne in the translucent base body. Thereby, the generation of convection is desirably promoted in the air flow space.

(6) In the lighting apparatus described in (1) to (5), the air flow space is provided between the lamp and the translucent base body and between the translucent base bodies themselves, and is formed such that the air can flow into and out of the lamp. Therefore, the photocatalytic reaction layer and the air are easily brought into contact with each other. In such configuration, the air flows into and out of the lamp from the lower side toward the upper side of the lighting apparatus by updraft action generated by the infrared light-absorbing function.

(7) In the lighting apparatus described in (1) to (6), a visible light transmitted through the translucent base body in the light emitted from the lamp is used for lighting, which is desirable because the use of the fluorescent material is eliminated.

(8) In the lighting apparatus described in (1) to (7), an outermost base body among the translucent base bodies can be used for decoration or advertisement, which is desirable because aesthetic and advertising effects can be exerted.

In the present invention, the "full spectrum light" usually means light having a continuous spectrum and at least two kinds of wavelength bands. However, as described later, the "full spectrum light" which is of a target of the present invention shall also mean light having a peak in a discontinuous spectrum with at least two kinds of wavelength bands.

In the present invention, the "ultraviolet spectrum" means a wavelength range including, as an upper limit, a short wavelength end of 360 to 400 nm of the visible light and a lower limit of about 10 nm, the "visible spectrum" means a wavelength range including a lower limit of about 360 to about 400 nm and an upper limit of about 760 to about 830 nm, and the "infrared spectrum" means a wavelength range including, as a lower limit, a long wavelength end of 760 to 830 nm of the visible light and having an upper limit of about 1 mm.

In accordance with the lighting apparatus of the present invention, the ultraviolet light, visible light and infrared light each having different wavelengths can effectively be utilized according to characteristics such as an air purifying function of ultraviolet light, a lighting function of visible light, and a heating function of infrared light to hereby save the optical energy emitted from the lamp.

In the case of the use of the plural translucent base bodies, since the outermost base body can be used for decoration or advertisement, the lighting apparatus of the present invention can be utilized in the advertising etc.

The forced convection is generated in the air flow space by the heating of the infrared light emitted from the lamp in addition to the heating of the optical energy emitted from the lamp, so that the air can sufficiently be ventilated to promote an air purifying effect.

### BRIEF DESCRIPTION OF THE DRAWINGS

Figs. 1(a) and 1(b) are views schematically showing a configuration example of a lighting apparatus of the present invention, in which Fig. 1(a) is a perspective view of the lighting apparatus, and Fig. 1 (b) is a front view of the lighting apparatus.
Figs. 2(a), 2(b) and 2(c) are views each schematically showing an example of a translucent base body having an infrared light-absorbing function, in which Fig. 2 (a) is a view showing the case where an infrared light-absorbing material is applied on a surface of the translucent base body, Fig. 2 (b) is a view showing the case where photocatalytic reaction layers are formed in base bodies which absorb infrared light, and Fig. 2(c) is a view showing the case where the base body which absorbs the infrared light is inserted between other base bodies.
Figs. 3(a) and 3(b) are photographs each showing an external appearance of a decorated lighting apparatus of the present invention, in which Fig. 3(a) is a photograph showing a front face of the decorated lighting apparatus, and Fig. 3(b) is a photograph taken from obliquely above.
Fig. 4 is a view showing a configuration of a lighting apparatus used in a first example of the present invention.
Fig. 5 is a view showing a configuration of a lighting apparatus used in a second example of the present invention.
Fig. 6 is a view showing a configuration of a lighting apparatus used in a third example of the present invention.

### BEST MODES FOR CARRYING OUT THE INVENTION

A lighting apparatus according to the present invention is characterized in that a full spectrum light emitting lamp and one or more translucent base bodies surrounding the lamp are disposed in the lighting apparatus, the one or more translucent base bodies having a photocatalytic reaction layer which bears a photocatalyst made of a titanium dioxide thin film therein, or the one or more translucent base bodies having the photocatalytic reaction layer which bears the photocatalyst made of the titanium dioxide thin film therein and having infrared light-absorbing functions, and a space through which air can flow is formed between the lamp and the translucent base body. The lighting apparatus of the present invention can exert the excellent air purifying function by adopting these configurations.

In an embodiment of the present invention, the wavelength of the light emitted from the lamp is not limited to the full spectrum light having a simply continuous spectrum, but the wavelength of the light can include, even if discontinuously, at least the ultraviolet light of the ultraviolet spectral range and the visible light of the visible spectral range or at least the infrared light of the infrared spectral range and the visible light of the visible spectral range. The infrared light of the infrared spectral range can be included in addition to the ultraviolet light of the ultraviolet spectral range and the visible light of the visible spectral range.

A configuration of the lighting apparatus of the present invention will be described below with reference to the drawings.

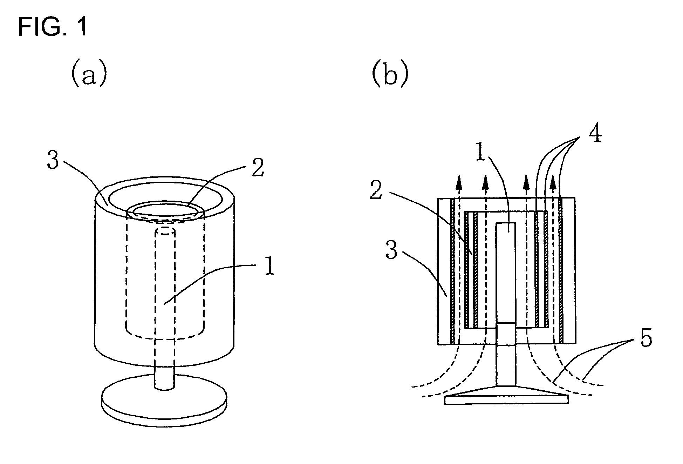

Figs. 1(a) and 1(b) are views each schematically showing a configuration example of a lighting apparatus of the present invention, in which Fig. 1(a) is a perspective view of the lighting apparatus, and Fig. 1(b) is a front view of the lighting apparatus. In the lighting apparatus shown in Fig. 1, a full spectrum light emitting lamp 1 is surrounded by an internal cylinder 2 and an external cylinder 3 which are of the translucent base bodies, and photocatalytic reaction layers 4 are formed in an inner and outer surfaces of the internal cylinder 2 and in an inner surface of the external cylinder 3. Each photocatalytic reaction layer 4 bears a photocatalyst made of a titanium dioxide thin film.

If needed, the internal cylinder 2 and the external cylinder 3 are formed by the translucent base body made of a material having an infrared light-absorbing property, and a space through which air can flow is formed between the lamp 1 and the internal cylinder 2 and between the internal cylinder 2 and the external cylinder 3.

In the above configuration, the full spectrum light emitted from the lamp 1 is transmitted through the internal cylinder 2 in which the photocatalytic reaction layers 4 are formed, whereby the titanium dioxide photocatalyst is activated to exert strong oxidizing power.

In the case where the internal cylinder 2 is formed by the translucent base body made of a material having an infrared light-absorbing property, the light is converted into thermal energy, and air convection is generated in the direction 5 of broken-line arrows in Fig. 1 by the heat emitted from the base body surface. Therefore, the air flow space is sufficiently ventilated to promote the air purifying effect.

Similarly, in the external cylinder 3, the strong oxidizing power is exerted by the titanium dioxide, and the air convection is generated by the heat emitted from the base body surface when the external cylinder 3 is formed by the translucent base body made of the material having the infrared light-absorbing property. Therefore, the air purifying effect is further promoted by a synergetic effect with a reaction generated by the internal cylinder 2. The light which is not utilized for the photocatalysis effect and heating effect, that is, the light which is transmitted through the internal cylinder 2 and external cylinder 3 is used for lighting.

As described above, the "full spectrum light" employed in the lighting apparatus of the present invention means light having various wavelength bands. The lamp which emits the full spectrum light is not limited to a particular type. For example, True Lite which emits the light close to the sun light can be used as the lamp.

However, in the lighting apparatus of the present invention, it should be noted that the lamp which emits the continuous spectrum light or the lamp which emits the light having at least two kinds of any wavelength bands does not always achieve the air purifying function of the present invention.

That is, in the lighting apparatus of the present invention, in order to secure the air purifying function of the titanium dioxide and lighting function as the embodiment, it is defined that the lamp used as the light source emits at least the ultraviolet light of the ultraviolet spectral range and the visible light of the visible spectral range or at least the infrared light of the infrared spectral range and the visible light of the visible spectral range.

Furthermore, in another embodiment, in order to secure the heating function in addition to the above-described functions, the lamp used as the light source emits the ultraviolet light of the ultraviolet spectral range, the visible light of the visible spectral range, and the infrared light of the infrared spectral range.

In the lighting apparatus of the present invention, the photocatalytic reaction layer desirably exhibits photocatalysis activity by the ultraviolet light as a reaction characteristic of the photocatalytic reaction layer formed in the translucent base body. Therefore, biologically harmful ultraviolet light can effectively be used.

With reference to another reaction characteristic of the photocatalytic reaction layer, the photocatalytic reaction layer formed in the translucent base body desirably exhibits the photocatalysis activity by the ultraviolet light and visible light, which allows the ultraviolet intensity to be supplemented by the visible light.

In the lighting apparatus of the present invention, the translucent base body having the infrared light-absorbing function is an essential component. The infrared light-absorbing function can be exerted by the infrared light-absorbing material applied on a surface of the translucent base body, or by having the infrared light-absorbing function borne by the translucent base body.

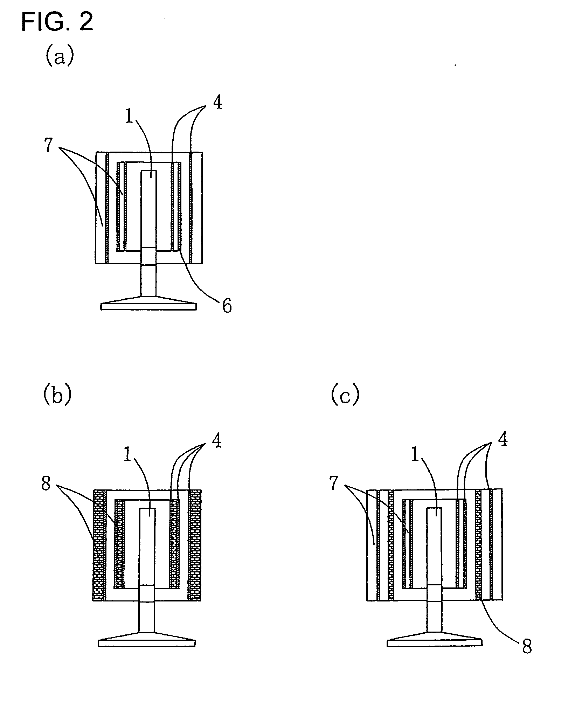

Figs. 2(a), 2(b) and 2(c) are views each schematically showing an example of the translucent base body having the infrared light-absorbing function, in which Fig. 2 (a) is a view showing the case where the infrared light-absorbing material is applied on a surface of the translucent base body, Fig. 2 (b) is a view showing the case where photocatalytic reaction layers are formed in base bodies which absorb infrared light, and Fig. 2 (c) is a view showing the case where the base body which absorbs the infrared light is inserted between other base bodies.

In the case where the infrared light-absorbing function is exerted by the infrared light-absorbing material applied on the surface of the translucent base body, as shown in Fig. 2(a), the lamp 1 is surrounded by transparent glass cylinders 7, the photocatalytic reaction layers 4 which bear the photocatalyst made of the titanium dioxide thin film are formed in the inner surfaces of the internal cylinder and external cylinder, and an infrared light-absorbing material 6 is applied on the outer surfaces of the internal cylinder and external cylinder.

In such a configuration, the light emitted from the lamp 1 is converted into the thermal energy by the infrared light-absorbing material 6, and the forced convection is generated by the heat emitted from the base body surface, thereby sufficiently ventilating the air flow space.

With reference to the embodiment in which the infrared light-absorbing function is exerted by having the function borne by the translucent base body, as shown in Fig. 2(b), the lamp 1 is surrounded by glass cylinders 8 having the infrared light-absorbing function, and the photocatalytic reaction layers 4 can be formed on surfaces of the glass cylinders 8.

Further, as shown in Fig. 2(c), the lamp 1 is surrounded by the transparent glass cylinders 7 in which the photocatalytic reaction layers 4 are formed, and the glass cylinder 8 having the infrared light-absorbing function can be inserted between the internal cylinder and the external cylinder.

In both the cases shown in Figs. 2(b) and 2(c), the light emitted from the lamp 1 is converted into the thermal energy by the glass cylinders 8 having the infrared light-absorbing function, and the forced convection is generated by the heat emitted from the base body surface, which allows the air flow space to be sufficiently ventilated.

The air flow space is an essential structural element in the lighting apparatus of the present invention. The air flow space is desirably provided between the lamp and the translucent base body and between the translucent base bodies themselves such that the air can flow into and out of the lamp. In such a configuration, it is not necessary to provide an air flow path separately. Therefore, the lighting apparatus can be easily produced, and the air and the photocatalytic reaction layer are easily brought into contact with each other. This configuration causes the flowing of the air into and out of the lamp to proceed from the lower side toward the upper side by updraft action generated by the infrared light-absorbing function.

In the lighting apparatus of the present invention, it is desirable to use, as the lighting, the visible light passed through the translucent base body in the light emitted from the lamp. Therefore, since a fluorescent material is not needed as an essential structural element, a usable life of the photocatalyst is not influenced by a life-time of the fluorescent material, and the waste of the photocatalyst can be prevented.

In the lighting apparatus of the present invention, an outermost base body of the translucent base bodies can be used for decoration and advertisement.

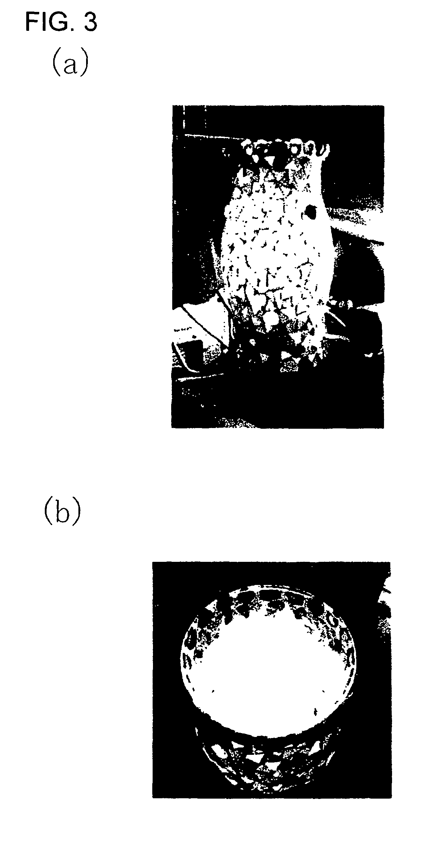

Figs. 3(a) and 3(b) are photographs each showing an external appearance of the decorated lighting apparatus of the present invention, in which Fig. 3(a) is a photograph showing a front face of the decorated lighting apparatus, and Fig. 3(b) is a photograph taken from obliquely above. While the lighting apparatus of the present invention is characterized by having the excellent air purifying function, as shown in Fig. 3, the lighting apparatus can also be used as a very beautiful interior by decorating the outermost base body of the translucent base bodies.

When a lighting apparatus of the present invention is used for interiors, it is advantageous that cleaning is easily performed, in addition to indoor air purification and deodorization effects. Additionally, the lighting apparatus can be used for an advertising display and an information sign, and fouling due to dusts and exhaust gases is prevented, so that maintenance management costs can be reduced.

When only the internal cylinder bears the photocatalyst, it is avoided to discard the photocatalyst when replacing or discarding the external cylinder used as the decoration and advertisement. In the case where the internal cylinder is eliminated, needless to say, obviously the photocatalytic reaction layer can be formed in the inner surface of the external cylinder.

### EXAMPLES

In order to confirm the effect of the air purifying property borne by the lighting apparatus of the present invention, an acetaldehyde decomposition test is performed to evaluate air purifying performance.

### (First Example)

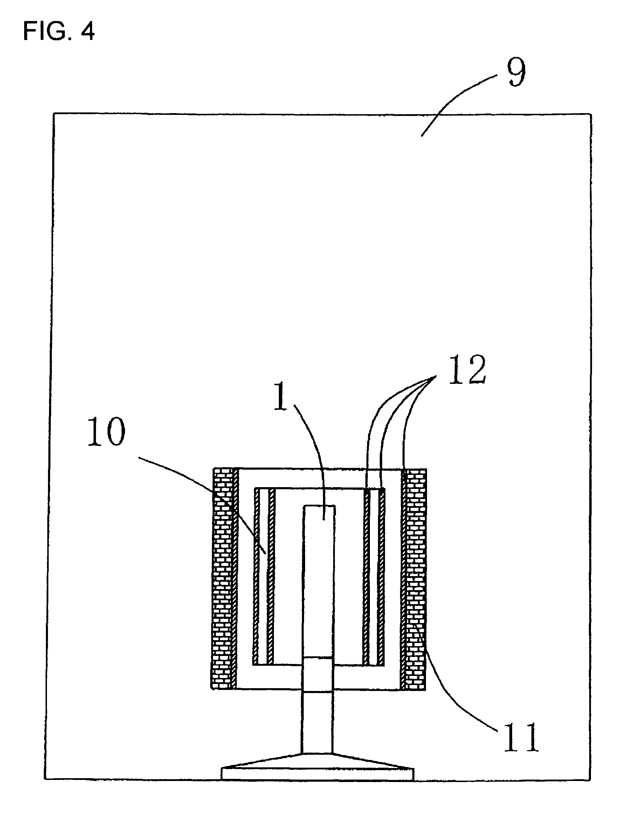

Fig. 4 is a view showing a configuration of a lighting apparatus used in a first example of the present invention. In the first example, two translucent base bodies 10 and 11 surrounding the lamp 1 were disposed in a test container 9. SPIRAL VITA LITE PLUS (20W) was used as the lamp 1. SPIRAL VITA LITE PLUS has a radiation spectrum close to the sun light, and emits the ultraviolet light, the visible light, and the infrared light. The ultraviolet light quantity was about 400 µW/cm2 in the side of the lamp at a 40-mm position away from the center of the lamp 1, and the ultraviolet light quantity was about 200 µW/cm² in the side of the lamp at a 60-mm position away from the center of the lamp 1.

A transparent glass cylinder having an external diameter of 80 mm, a height of 240 mm, and a thickness of 2 mm was used as the internal cylinder 10 of the two translucent base bodies. Photocatalyst titanium oxides 12 were coated on both the inner and outer surfaces of the internal cylinder 10 by a Chemical Vapor Deposition (CVD) method. A glass cylinder, which has the infrared light-absorbing property, with an external diameter of 80 mm and a height of 300 mm was used as the external cylinder 11. The photocatalyst titanium oxide 12 was coated on the inner surface of the external cylinder 11, and decoration pictures were provided in the outer surface by color glass.

The lighting apparatus having the excellent air purifying property was placed in the sealable stainless-steel test container 9 with an internal diameter of 550 mm, a height of 900 mm, and a volume of about 200L, and the air in the stainless-steel test container 9 was replaced with synthetic air containing 25-ppm acetaldehyde.

The lamp 1 was retained for one hour while not lit, and an acetaldehyde concentration was measured in the stainless-steel test container 9. The test container 9 had the acetaldehyde concentration of 22 ppm. The lamp 1 was retained for one more hour to measure the acetaldehyde concentration inside the test container 9, with the result of showing the acetaldehyde concentration of 22 ppm. Therefore, it was confirmed that the acetaldehyde concentration is not changed when the lamp 1 is not lit.

The lamp 1 of the lighting apparatus was lit, and a time-dependent change of the acetaldehyde concentration was measured inside the stainless-steel test container 9 by gas chromatography.

The acetaldehyde concentration inside the test container 9 was 19 ppm after one hour elapsed since the lamp 1 was lit. The acetaldehyde concentration inside the test container 9 was 10ppm after five hours elapsed since the lamp 1 was lit. Further, the acetaldehyde concentration inside the test container 9 was 4.5 ppm after ten hours elapsed since the lamp 1 was lit, and the acetaldehyde concentration became 1 ppm or less after 20 hours elapsed since the lamp 1 was lit. Therefore, it could be confirmed that the lighting apparatus of the present invention can exhibit the excellent purifying effect.

This is attributed to the fact that the excellent purifying effect is exerted by the generation of the effective convection caused by a difference in air temperature between the outside atmosphere and the air flow space. Due to the effect of utilizing the glass cylinder having the infrared light-absorbing property, the external cylinder glass side surface had the temperature of about 38°C when the lamp is lit, while room temperature was 25°C. Since illuminance was 1000 lux or more in the neighborhood of the external cylinder glass side surface, and therefore, it could be confirmed that the sufficient brightness is secured as interior lighting.

### (Second Example)

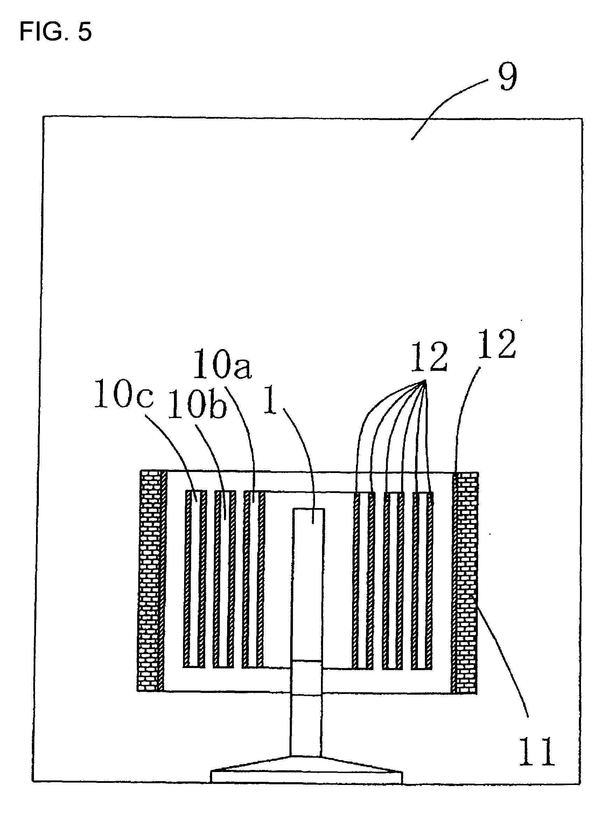

Fig. 5 is a view showing a configuration of a lighting apparatus used in a second example of the present invention. In the second example, as translucent base bodies, three internal cylinders 10a, 10b and 10c and an external cylinder 11 which surround the lamp 1 were provided, and the lighting apparatus was placed in the test container 9.

As shown in Fig. 5, the lighting apparatus had a triple-wall structure in which transparent glass cylinders were used. The transparent glass cylinders had the same height of 240 mm, and the internal cylinder 10a, the internal cylinder 10b and the internal cylinder 10c had the external diameters of 80 mm, 120 mm and 150 mm, respectively. The external cylinder 11 was formed of a glass cylinder having the infrared light-absorbing property, with the external diameter of 180 mm and the height of 300 mm. Similarly to the first example, the photocatalyst titanium oxides 12 were coated by the CVD method on both the inner and outer surfaces of the internal cylinders 10a, 10b and 10c and on the inner surface of the external cylinder 11.

The acetaldehyde decomposition test was performed in the method similar to that of the first example. The inside of the stainless-steel test container 9 showed the acetaldehyde concentration of 20 ppm when the lamp 1 was retained while not lit. The inside of the test container 9 showed the acetaldehyde concentration of 15 ppm after one hour elapsed since the lamp 1 was lit. Further, the inside of the test container 9 showed the acetaldehyde concentration of 4.3 ppm after five hours elapsed since the lamp 1 was lit, and showed the acetaldehyde concentration of 1 ppm or less after 10 hours elapsed.

In the second example, it was confirmed that the purifying effect is more significant than that of the first example. The lighting apparatus of the second example differed from that of the first example in that the internal cylinder had the triple-wall structure. Accordingly, in the lighting apparatus of the present invention having the excellent air purifying property, it was confirmed that the purifying effect is improved with increasing surface area of photocatalytic reaction layers contacting the ambient air.

### (Third Example)

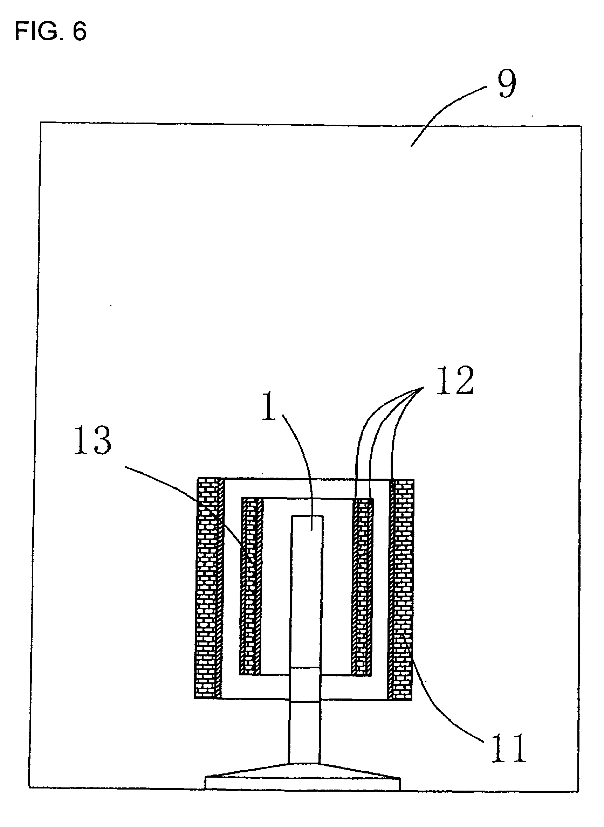

Fig. 6 is a view showing a configuration of a lighting apparatus used in a third example of the present invention. As shown in Fig. 6, the lighting apparatus of the third example was similar to that of the first example except that glass having the infrared light-absorbing property was used for an internal cylinder 13. That is, the photocatalyst titanium oxides 12 were coated by the CVD method on both the inner and outer surfaces of the internal cylinder 13 and on the inner surface of the external cylinder 11.

The acetaldehyde decomposition test was performed in the method similar to that of the first example. The inside of the stainless-steel test container 9 showed the acetaldehyde concentration of 22 ppm when the lamp 1 was retained while not lit. The inside of the test container 9 showed the acetaldehyde concentration of 18 ppm after one hour elapsed since the lamp 1 was lit. Further, the inside of the test container 9 showed the acetaldehyde concentration of 8 ppm after five hours elapsed since the lamp 1 was lit, showed the acetaldehyde concentration of 3 ppm after 10 hours elapsed, and showed the acetaldehyde concentration of 1 ppm or less after 16 hours elapsed.

In the third example, it was confirmed that the purifying effect is more significant than that of the first example. The lighting apparatus of the third example differed from that of the first example in that the internal cylinder 13 was made of the glass having the infrared light-absorbing property. Accordingly, it was confirmed that the forced convection generated by the heat emitted from the base body surface is promoted by the use of the infrared light-absorbing base body, and the purifying effect of the lighting apparatus of the present invention is further improved by increasing contact efficiency between the photocatalyst and the ambient air.

### INDUSTRIAL APPLICABILITY

According to the lighting apparatus of the present invention, the ultraviolet light, the visible light and the infrared light can effectively be utilized according to the characteristics such as the air purifying function of the ultraviolet light, the lighting function of the visible light, and the heating function of the infrared light to thereby save the optical energy emitted from the lamp. In the case where the plural translucent base bodies are used, the outermost base body can be used as the decoration and advertisement, and the lighting apparatus of the present invention can be utilized in the advertising.

The forced convection is generated in the air flow space by heating effect of the infrared light emitted from the lamp in addition to the heating effect of the optical energy emitted from the lamp, so that the air can sufficiently be ventilated to promote the air purifying effect. Therefore, the lighting apparatus of the present invention can be applied to interiors as being excellent in the indoor air purification and deodorization effects, and to the advertising display and information sign which can prevent fouling due to dusts and exhaust gases.

## Claims

1. A lighting apparatus having an excellent air purifying property, **characterized in that:**
a full spectrum light emitting lamp and one or more translucent base bodies surrounding the lamp are disposed in the lighting apparatus, the one or more translucent base bodies having a photocatalytic reaction layer which bears a photocatalyst made of a titanium dioxide thin film therein, or the one or more translucent base bodies having the photocatalytic reaction layer which bears the photocatalyst made of the titanium dioxide thin film therein and having infrared light-absorbing functions; and
a space is formed between the lamp and the translucent base body to enable air to flow therethrough.

2. The lighting apparatus having an excellent air purifying property according to claim 1, **characterized in that** one or more translucent base bodies having the infrared light-absorbing functions are disposed so as to surround the lamp.

3. The lighting apparatus having an excellent air purifying property according to claim 1 or 2, **characterized in that** the lamp emits light which is included in at least ultraviolet spectral and visible spectral wavelength ranges.

4. The lighting apparatus having an excellent air purifying property according to claim 1 or 2, **characterized in that** the lamp emits light which is included in at least infrared spectral and visible spectral wavelength ranges.

5. The lighting apparatus having an excellent air purifying property according to claim 1 or 2, **characterized in that** the lamp emits light which is included in ultraviolet spectral, visible spectral and infrared spectral wavelength ranges.

6. The lighting apparatus having an excellent air purifying property according to any one of claims 1 to 5, **characterized in that** the photocatalytic reaction layer exerts photocatalysis activity by an ultraviolet light in the light emitted from the lamp.

7. The lighting apparatus having an excellent air purifying property according to any one of claims 1 to 5, **characterized in that** the photocatalytic reaction layer exerts photocatalysis activity by an ultraviolet light and a visible light in the light emitted from the lamp.

8. The lighting apparatus having an excellent air purifying property according to any one of claims 1 to 7, **characterized in that** the infrared light-absorbing function is exerted by an infrared light-absorbing material applied on a surface of the translucent base body.

9. The lighting apparatus having an excellent air purifying property according to any one of claims 1 to 7, **characterized in that** the infrared light-absorbing function is exerted by a function borne in the translucent base body.

10. The lighting apparatus having an excellent air purifying property according to any one of claims 1 to 9, **characterized in that** the air flow space is provided between the lamp and the translucent base body and bet ween the trans lucent base bodies themselves, and is formed such that the air can flow into and out of the lamp.

11. The lighting apparatus having an excellent air purifying property according to claim 10, **characterized in that** the air flows into and out of the lamp from the lower side toward the upper side of the lighting apparatus by updraft action generated by the infrared light-absorbing function.

12. The lighting apparatus having an excellent air purifying property according to any one of claims 1 to 11, **characterized in that** a visible light transmitted through the translucent base body in the light emitted from the lamp is used for lighting.

13. The lighting apparatus having an excellent air purifying property according to any one of claims 1 to 12, **characterized in that** an outermost base body among the translucent base bodies is used for decoration or advertisement.
